# EUROPEAN PATENT APPLICATION

(11) **EP 4 043 435 A1**
(43) Date of publication of application: **17.08.2022**
(21) Application number: 20874025.8
(22) Date of filing: 11.09.2020
(51) Int. Cl.: C07D 209/12, C07H 15/26, G01N 33/52, A61K 49/00

(54) **COMPOUND FOR DETECTING SENESCENT CELLS AND USE THEREOF**

(30) Priority: 10.10.2019 ES 201930893
(71) Applicant: Universitat Politècnica de València, 46022 Valencia (ES); CENTRO DE INVESTIGACION PRINCIPE FELIPE, 46012 Valencia (ES)
(72) Inventor: LOZANO TORRES, Beatriz, 46022 Valencia (ES); BLANDEZ BARRADAS, Juan Francisco, 46022 Valencia (ES); MARTÍNEZ MAÑEZ, Ramón, 46022 Valencia (ES); GALIANA GUILLEM, Irene, 40622 Valencia (ES); GARCÍA FERNÁNDEZ, Alba, 46022 Valencia (ES); SANCENÓN GALARZA, Félix, 46022 Valencia (ES); ORZÁEZ CALATAYUD, Mar, 46012 Valencia (ES)
(74) Representative: Pons
(86) International application number: PCT/ES2020/070547
(87) International publication number: WO 2021/069773

(57) **Abstract**

The invention relates to a detection compound or probe that exhibits high diuretic activity and comprises a signalling unit (fluorophore) bearing negatively charged functional groups (anionic groups). The recognition unit, which consists of a saccharide, is bound to the signalling unit by way of a direct covalent bond. The invention also relates to the use of the compound as a probe for detecting senescent cells in urine.

## Description

The present invention relates to a compound, also referred to as a diagnostic tool or probe, for detecting senescent cells through urine, thereby achieving a quantification of cellular senescence. The present invention is, therefore, comprised in the field of medicine, and more particularly, diagnostic tools.

### BACKGROUND OF THE INVENTION

The primary role of cellular senescence is to prevent the proliferation of damaged or stressed cells and to subsequently eliminate them. Furthermore, cellular senescence acts as a trigger for the activation of damaged tissue repair and regeneration processes or during the embryonic process, which indicates that the presence of senescent cells may also represent a benefit for the biological system. However, these tissue repair processes may become inefficient when cell damage persists or during ageing. This insufficiency allows senescent cells to accumulate on damaged tissues or organs, which promotes local inflammations, the ageing and destruction of tissues, the formation of tumours and even the generation of metastasis. It has been demonstrated that the selective elimination of senescent cells by means of cellular apoptosis (senolysis) promotes the improvement of a wide variety of diseases associated with the presence of senescent cells, reversing the symptoms thereof and improving the quality of life and life expectancy of individuals. In fact, studies have been carried out in murine models which have corroborated the efficacy of senolysis processes for the elimination and improvement of disease symptoms such as renal dysfunction, pulmonary fibrosis, hypertrophy or sarcopenia. The literature describes that certain small molecules have therapeutic effects for multiple diseases, and this pharmacological activity is based on the ability to promote senolysis. Based on these same principles, there are studies which prove that senolysis may exhibit the same efficacy for the treatment of diseases related to cellular ageing.

In the field of cellular senescence, the development of new methods for achieving the detection of senescent cells as soon as they appear in the organism, as well as at when they start to accumulate, is extremely important. However, most of the methods described today cannot be applied for directly detecting senescent cells and virtually none of them can be used in *in vivo* models. Based on the foregoing, the need for the development of new systems or methods for detecting senescent cells that can be used as diagnostic tools has been clearly shown, and even more important is being able to achieve a method which allows the degree of cellular senescence to be quantified and enables the monitoring of treatments with senotherapeutics in the different diseases associated with the accumulation of senescent cells.

Patent document US2016229840 describes fluorogenic compounds designed such that, upon a chemical event, compounds capable of emitting fluorescence are generated. Also described are uses of the fluorogenic compounds to monitor the presence and/or level of various analytes. Compared to patent document US2016229840, which describes probes with photochemical properties similar to those described in the present invention, the probes proposed in the present invention present diuretic characteristics which allow the measurement of analytes, in this case the detection of senescent cells, in a non-invasive manner through urine. Said diuretic property implies that the probes are rapidly expelled from the body, preventing the bioaccumulation thereof and allowing the storage of samples for the subsequent measurement thereof if said measurement cannot be performed *in situ.*

Patent document ES2710322 (T3) is based on compounds useful for visualizing cellular senescence, the preparation and the use thereof. In particular, this invention relates to new fucose and amino-quinoline derivatives useful as senescence tracers and the preparation thereof. Patent document ES2710322 (T3) uses probes for detecting senescence comprising two types of markers, a fluorogenic marker and a second radioisotope marker. Senescence is detected *in vitro* by fluorescence by means of techniques based on flow cytometry, and techniques of this type cannot be used for systems *in vivo.* Furthermore, senescence is detected *in vivo* with halogen radioisotopes. The present invention proposes a method for detecting senescence *in vivo* based on fluorescence, the simplest method, which uses much more common and affordable measurement equipment, techniques which can be used by non-qualified personnel. Furthermore, even though the two are non-invasive techniques in both cases, the present invention proposes measuring senescence through urine due to the intrinsic characteristics of the probes; this allows the storage of samples for a subsequent measurement in the event that direct measurements of the samples cannot be performed. Lastly, compared to the probes described in patent document ES2710322 (T3) which exhibit complex synthetic routes, in the present invention the described synthetic route consists of two steps, starting directly from low-cost commercial compounds.

To that end, the present invention proposes a new tool (probe) for non-invasively detecting cellular senescence in subjects through urine.

### DESCRIPTION OF THE INVENTION

In a first aspect, the present invention relates to a compound characterized in that it comprises:
- a labelling unit which consists of a fluorophore comprising at least one anionic group and
- a recognition unit which consists of a saccharide,
wherein the labelling unit is bound directly and covalently to the recognition unit.

The direct covalent bond implies the non-existence of linking groups or "linkers" between the labelling unit and the recognition unit. Therefore, the compound of formula: 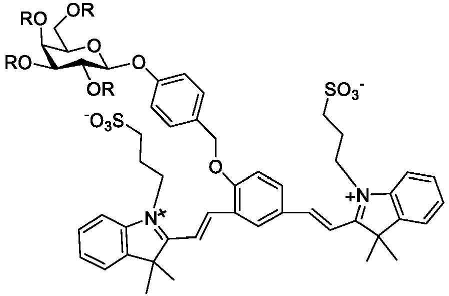 wherein R is H or an acetyl group (CH₃-CO-), would be excluded from the scope of the present invention, since there is more than one linking group between the monosaccharide and the fluorophore.

The term "saccharide" refers to a carbohydrate comprising, or consisting of, at least one monosaccharide (basic sugar unit), wherein the monosaccharides forming the saccharide are bound to one another by glycosidic bonds. In the present invention, the term monosaccharide includes the basic sugar unit comprising -OH groups (for example, glucose, galactose, fructose, etc.), as well as derivatives thereof. These derivatives are obtained by substitution of one of the hydroxyl groups with another functional group, or by oxidation or reduction of one of the carbon atoms of the original monosaccharide. The derivatives can be, for example, amino sugars, in which at least one hydroxyl group of the monosaccharide of origin is substituted with an amino group; sugar acids resulting from the oxidation of a carbon atom of the monosaccharide of origin to a carboxyl group; deoxysugars, in which one of the hydroxyl groups of the monosaccharide of origin is substituted with a hydrogen atom; sugar phosphates, which are phosphorylated sugars (bound to a phosphate group by means of an ester bond) in one of the hydroxyl groups, monosaccharides obtained by substitution of at least one -OH group with an -O', wherein R' is selected from: acetyl (Ac: CH₃-CO-), C1-C8 alkyl, arylalkyl, aryl and heteroaryl. In this latter case, the monosaccharide can have several or all of the substituted -OH groups. Preferably, the monosaccharide derivatives are those which in which all the -OH are substituted with -OAc. Included among the monosaccharides are the different anomeric forms thereof: α (alpha) and β (beta), as well as their Dextro (D) and Levo (L) configurations.

The term "C1-C8 alkyl" refers, in the present invention, to linear or branched aliphatic chains, having 1 to 8 carbon atoms, for example, methyl, ethyl, n-propyl, i-propyl, n-butyl, tert-butyl, sec-butyl, n-pentyl, n-hexyl, etc. Preferably, the alkyl group has between 1 and 4 carbon atoms. The alkyl groups can optionally be substituted with one or more substituents such as halogen, hydroxyl, azide, carboxylic acid or a substituted or unsubstituted group selected from amino, amido, carboxylic ester, ether, thiol, acylamino or carboxamido.

"Arylalkyl" is understood in the present invention to mean an aryl group bound to the rest of the molecule by an alkyl group. A non-limiting example of arylalkyl is a benzyl group.

The term "aryl" refers in the present invention to an aromatic carbocyclic chain, having 6 to 12 carbon atoms, where it can be a single or multiple ring, in this latter case having separated and/or condensed rings. A non-limiting example of aryl is a phenyl group. The aryl groups are, for example, but without being limited to, phenyl, naphthyl, diphenyl, indenyl, phenanthryl or anthracyl. The aryl radicals can optionally be substituted with one or more substituents.

The term "heteroaryl" refers, in the present invention, to aromatic cyclic (mono- or bicyclic) rings having between 5 and 10 links in which a carbon has been substituted with an electronegative element, such as sulphur, nitrogen, or oxygen. Preferably, the heteroaryl group has between 5 and 6 links. The aryl radicals can optionally be substituted with one or more substituents, such as alkyl, halogen, hydroxyl, alkoxide, thiol, nitro, amino, acylamino, cyano, carboxylate, carboxamide, carboxyl ester, or combinations of these groups.

The term "fluorophore" refers to a molecule capable of emitting electromagnetic radiation in response to the absorption of excitation radiation, with the wavelength of the emitted radiation being different from the wavelength of the excitation radiation, and wherein the emission of radiation persists only while the excitation radiation is maintained. This emission of radiation from the fluorophore of the invention gives rise to wavelengths that do not overlap with the self-emission coming from senescent cells existing in the biological samples.

The chemical bond between the signalling unit and the recognition unit is a covalent bond, preferably through an N, S, P or O atom of the signalling unit and the anomeric C atom of the saccharide, wherein the OH group of the anomeric carbon is substituted with the fluorophore. The signalling unit which consists of a fluorophore exhibits at least one anionic group (also referred to as diuretic unit), which is in charge of carrying out the diuretic function and of increasing the solubility of the compound so that it can be excreted through the urine. The anionic group can be selected from, for example and without being limited to, a carboxylate (-CO₂⁻), sulphonate (-SO₃⁻), sulphate (-SO₄⁻), halide (F^{-,} Cl⁻, Br⁻ and I⁻) group, or any anion with the ability to interact with Na⁺-K⁺-2Cl⁻cotransporter proteins located in the cells of the ascending limbs of the loops of Henle. In a preferred embodiment, the anionic groups bound to the fluorophore are sulfonate and/or carboxylate groups. In a more preferred embodiment, the signalling unit comprises two anionic groups, more preferably two sulfonate groups.

Preferably, the fluorophore forming the signalling unit is based on a cyanine structure exhibiting covalently bound anionic groups to confer diuretic properties thereto. More preferably, the fluorophore exhibits a structure (formula (I)) as defined below: 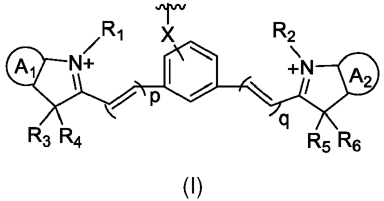 wherein A₁ and A₂ are independently aryl or heteroaryl groups (fused to the pyrrolinium ring, as shown in the structure),
*p* and *q* are independently an integer ranging between 1 and 4,
R₁ and R₂ are independently a -(CH₂)ₙ-Rₐ group, wherein:
   Rₐ is an anion selected from the list consisting of: -SO₃⁻ -CO₂⁻, -SO₄⁻, halide, n is an integer ranging between 0 and 8,
X is O, N, P or S, and
R₃, R₄, R₅, R₆ are selected independently from: C1-C8 alkyl, aryl and heteroaryl,
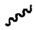 represents the point of the covalent bond with a carbon of a monosaccharide of the recognition unit.

The terms C1-C8 alkyl, aryl and heteroaryl are as defined above.

In a preferred embodiment of the fluorophore, A1 and A2 are independently phenyl.

In another preferred embodiment of the fluorophore, p and q are independently 1.

In another preferred embodiment of the fluorophore, R₁ and R₂ are independently a - (CH₂)ₙ -SO₃⁻ or -(CH₂)ₙ -CO₂⁻ group, n is between 1 and 8, more preferably n is 4.

In another preferred embodiment of the fluorophore, X is O.

In a preferred embodiment of the fluorophore, R₃, R₄, R₅, R₆ are independently methyl.

In another preferred embodiment, A1 and A2 are independently phenyl; p and q are independently 1 and X is O.

In another preferred embodiment, A1 and A2 are independently phenyl; p and q are independently 1; X is O and R₁ and R₂ are independently -(CH₂)₄ -SO₃⁻ or -(CH₂)₄ -CO₂.

In a preferred embodiment, the fluorophore has the following formula:

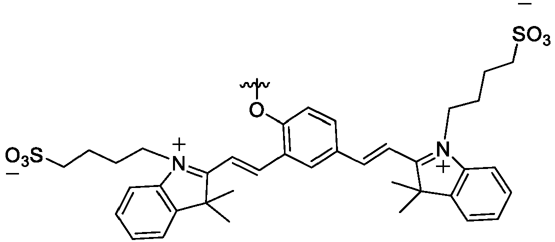

Preferably, the recognition unit is a saccharide comprising or consisting of between 1 and 100 monosaccharides.

In a more preferred embodiment, the saccharide comprises or consisting of between 1 and 50 monosaccharides, or even more preferably, between 1 and 20 monosaccharides.

In another preferred embodiment, 60 to 95 % of the monosaccharides that the saccharide comprises are galactose, fucose and/or derivatives thereof obtained by substitution of at least one -OH group, as described above in the definition of monosaccharide. More preferably, 60 to 95 % of the monosaccharides that the saccharide comprises are galactose, fucose and/or derivatives thereof wherein all the OH have been substituted with -OAc.

In another preferred embodiment, the monosaccharides that the saccharide comprises are galactose, fucose and/or derivatives thereof obtained by substitution of at least one -OH group, as described above in the definition of monosaccharide. More preferably, the monosaccharides that the saccharide comprises are galactose, fucose and/or derivatives thereof wherein all the OH have been substituted with -OAc.

In another preferred embodiment, the saccharide consists of a monosaccharide, preferably galactose, fucose or a derivative of any of them wherein all the OH have been substituted with -OAc.

Preferably, when reference is made in the present invention to galactose, it is to β -D-galactose, and when reference is made to fucose, it is preferably α-D fucose.

In a more preferred embodiment, the compound has the following formula: 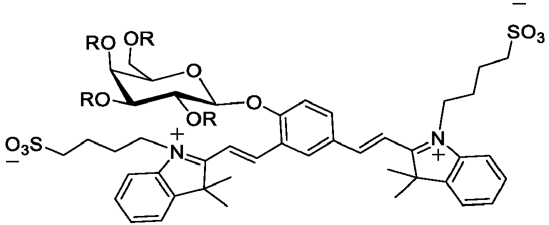 wherein R is H or an acetyl group (CH₃-CO-).

The senescent cells are characterised by an increase in the number and size of lysosomes causing the overexpression of lysosomal hydrolase enzymes, the most characteristic of which are the overexpression of activity of lysosomal enzyme β-galactosidase known as (SA-β gal) and the overexpression of activity of lysosomal enzyme α-fucosidase known as (SA-α-fuc).

In particular, in the present invention, activation of the compound is produced due to overactivity of the lysosomal enzyme; in the previous model, it would be produced by overactivity of β-galactosidase (SA-β-gal) associated with the presence of cellular senescence. This activity performs hydrolysis between the signalling unit and the recognition unit of the compound of the present invention. The disconnection of the recognition unit produces activation of the signalling unit. This activation is reflected by the occurrence of a noticeable fluorescence emission. Lastly, the anionic groups bound to the signalling unit exhibit high diuretic activity which allows the expulsion of the compound through urine. Since the diuretic unit is directly bound to the signalling unit, it allows the expulsion of both the activated and the deactivated compound, however, only the activated compound presents a fluorescence signal.

The anionic groups (sulfonates or carboxylates preferably) are used as triggers of diuretic activity by interacting on Na⁺-K⁺-2Cl⁻ cotransporter proteins located in the cells of the ascending limbs of the loops of Henle.

These anionic groups are bound at the existing active sites for Cl⁻ by blocking the protein cotransport system. For the functional groups to be able to interact in the active centres of the proteins, these functional groups must exhibit a geometry and an electrostatic potential similar to that of the analyte. To that end, the presence of the anionic groups plays a major role in the diuretic process.

The anionic groups present in the diuretic unit of the compound are bound to the Cl⁻recognition positions of the cotransport proteins and cause the high diuretic activity exhibited by the compound.

The compound of the present invention is, therefore, an easy-to-synthesise molecule which is initially in an "off" form. This compound must be administered in a simple manner, undergoing a transformation in the presence of senescent cells which allows the detection thereof by fluorescence both in the urinary system and after miction. The intensity of fluorescence observed in the urine samples is proportional to the amount of senescent cells, which allows the quantification and following of the number of senescent cells. In addition, a small amount of the activated compound accumulates in those areas where activation is produced (where the senescent cells are located), so due to the high fluorescence emission exhibited by the signalling unit, this small amount is sufficient to locate the area exhibiting cells immersed in senescence processes.

With these characteristics, the proposed probes allow detecting senescent cells *in vivo* through fluorescence measurements of the urine both before and after miction, giving rise to a non-invasive and quantitative method for determining cellular senescence, all of which allows the use thereof for different applications of great interest, such as:
- Use in monitoring chemotherapy treatments. Some chemotherapy treatments are based on the use of senescence inducers or the use of other medical protocols for the purpose of carrying out the transformation of tumour cells into senescent cells and achieving detection of cell proliferation. Therefore, the compound of the invention can be used to monitor the formation of senescent cells *in vivo* after the use of chemotherapy, allowing the quantification of cellular senescence in a non-invasive manner through fluorescence measurements of urine.
- They can be used to obtain information in the diagnosis of diseases related to ageing where conventional treatments are based on the use of senolytics. With this new tool, the elimination of these senescent cells with the drop in fluorescence emission obtained in the measurements of urine can be monitored.
- Lastly, the variation of the recognition unit would allow the use of the same protocol for detecting and monitoring other diseases or pathologies.

The term *"in vivo"* detection in the present invention relates to a detection through urine samples whether in the urinary system or after miction, carried out after administering the compound of the invention. In the present invention, the term *"in vivo"* is used to indicate that biopsies need not be performed or damaged areas need not be extracted, unlike what is required with most of the described methods which require tissue or cell extractions to determine the presence of cellular senescence *in vitro.*

Another aspect of the invention relates to a composition comprising the compound of the invention and a pharmaceutically acceptable carrier.

In a preferred embodiment, the composition is a pharmaceutical composition.

The "carrier" is preferably an inert substance. The function of the carrier is to facilitate the incorporation of other compounds and to allow better dosing and administration or provide consistency and form to the composition. Therefore, the carrier is a substance which is capable of allowing a better dosing and administration or provides consistency and form to the composition. The "pharmaceutically acceptable carrier" must allow the activity of the compound, be compatible with same and not cause damage to the organisms to which it is administered.

The composition of the invention can be administered by means of any of the known manners of administration. In a preferred embodiment, the composition is configured to be able to be administered intravenously, intraperitoneally or orally. In this latter case, the compound can be coated with a polymer that releases its content once it has passed the stomach. More preferably, the composition is configured to be injected by intravenous or intraperitoneal route.

Another aspect of the invention relates to the use of the compound of the invention, or of the composition comprising same, such as a probe for detecting senescent cells in a urine sample in which said compound has previously been administered.

The terms "probe" or "diagnostic tool" are used as synonyms in the present invention and refer to a chemical compound capable of selectively detecting cellular senescence. The probe of the invention furthermore exhibits the following characteristics:
- minimal or zero toxicity;
- a drop in the fluorescence signal, or no fluorescence, compared with the unmodified signalling unit (fluorophore) up until the moment in which the rupture of the bond in the presence of senescent cells takes place due to high β-galactosidase, α-fucosidase or other lysosomal hydrolase activity;
- the probe is kept stable in its inactivated form (signalling unit and recognition unit bound together) until it is in the presence of senescent cells;
- the probe retains most of the physical and chemical characteristics which allow its use in a manner similar to that of the signalling unit (fluorophore).

Senescent cells are cells exhibiting cell cycle arrest, which prevents normal cell activity and cell division. These cells exhibit a flattened morphology in the cytoplasm of which the number and size of vacuoles have increased. Senescent cells exhibit a distinctive a phenotype and a modification of their genetic expression allowing their identification. Among the assays commonly used for their identification, the most important assay is the determination of overactivity of the β-galactosidase (β-gal) enzyme associated with senescence.

For use as a probe, the compound can be administered to a subject by means of any of the known manners of administration; preferably intravenously, intraperitoneally or orally.

The probe exhibits diuretic properties (diuretic probe) so, once administered, this probe is cleaved in the presence of senescent cells such that the signalling unit is excreted in urine and can be detected by fluorescence, revealing the existence of senescent cells. Then, for this purpose, the compound of the invention can be used as a diuretic probe (a probe with diuretic properties), the active form of which (once it is cleaved into the two units forming same) can be visualised by fluorescence in excreted urine, revealing the existence of cellular senescence. Similarly, when the probe is not activated, the diuretic unit carries out the elimination thereof, in this case no fluorescence is observed in urine. This means that the probe has not accumulated in the body and is rapidly expelled through the urinary system.

The use of the probe for detecting senescent cells through urine allows carrying out a quantitative study of the degree of cellular senescence present in the subject, in addition to obtaining information about the possible presence of diseases of the subject derived from the accumulation of said cells.

Another aspect of the invention relates to the compound of the invention for use in a method of detecting senescent cells through urine. More specifically, for or in, a method of detecting senescent cells in a urine sample of a subject to whom said compound has previously been administered.

Another aspect of the invention relates to a method of detecting senescent cells comprising the following steps:
- administering an effective amount of the compound of the invention to a subject,
- obtaining a urine sample after administering the compound,
- obtaining fluorescence data from the obtained urine sample.

In the present invention, the expression "effective amount" refers to that amount of the compound which, when administered, is sufficient for being detected by the urine once it has been hydrolysed in the organism.

Throughout the description and the claims, the word "comprises" and its variants do not intend to exclude other technical features, additives, components or steps. For those skilled in the art, other objects, advantages and features of the invention may be partially deduced from both the description and the embodiment of the invention. The following examples and figures are provided by way of illustration and are not intended to limit the present invention.

### BRIEF DESCRIPTION OF THE FIGURES

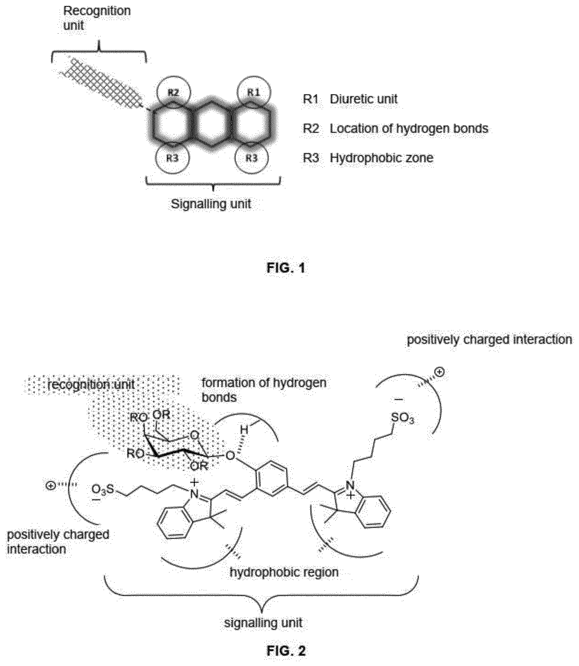

**FIG. 1****.** General structure of a probe in which the different units forming same can be seen: signalling unit including the diuretic units or groups and recognition unit.
**FIG. 2****.** Structure of Cy7-Gal, one of the possible probes (compounds of the present invention), in which the different groups, each of which has an individual function, are shown.

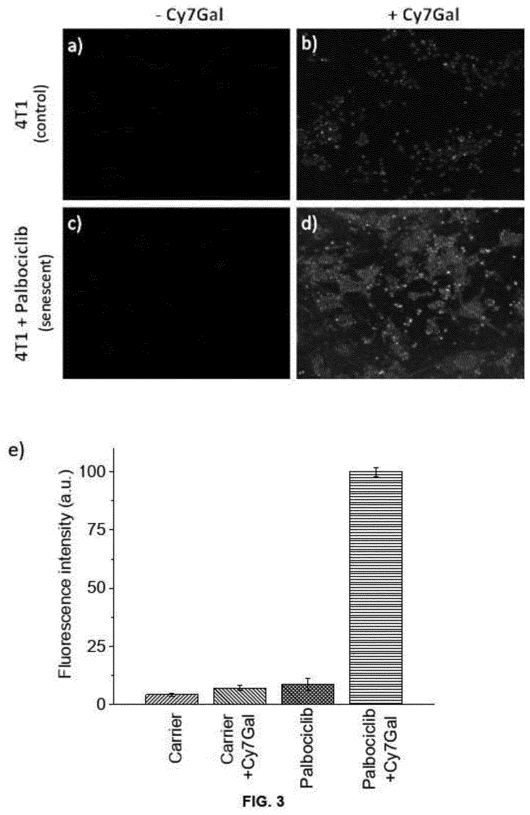

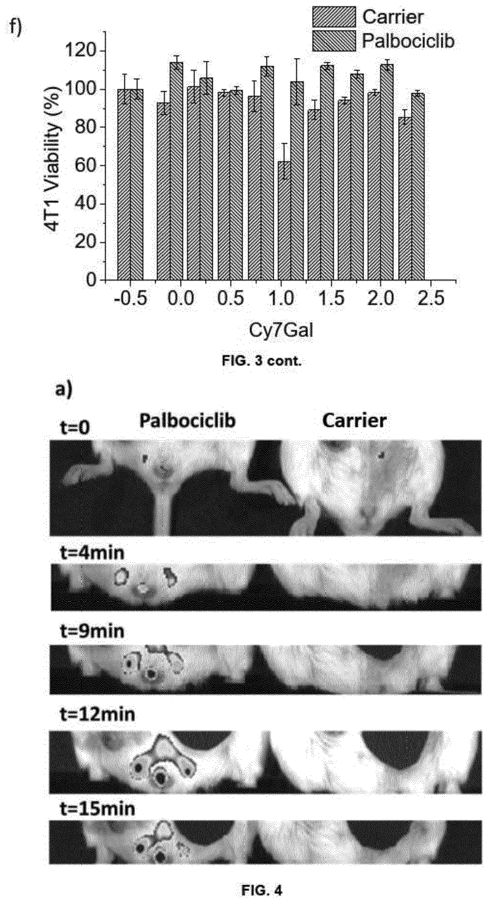

**FIG. 3****.** (a-d) Confocal microscopy images for 4T1 cells. (a,b) Images obtained for normal 4T1 cells. (c,d) Images of 4T1 cells treated with Palbociclib to induce cellular senescence. (a,c) Images of control and senescent cells in the absence of probe Cy7-Gal. (b,d) Control and senescent cells after being incubated for 30 min in the presence of Cy7-Gal. e) Quantification of the fluorescence emission intensity for control 4T1 cells and 4T1 cells treated with Palbociclib (senescent) incubated in the absence or in the presence of probe Cy7-Gal, f) Viability of 4T1 control and senescent cells after incubation with Cy7-Gal at different concentrations after 24 h.

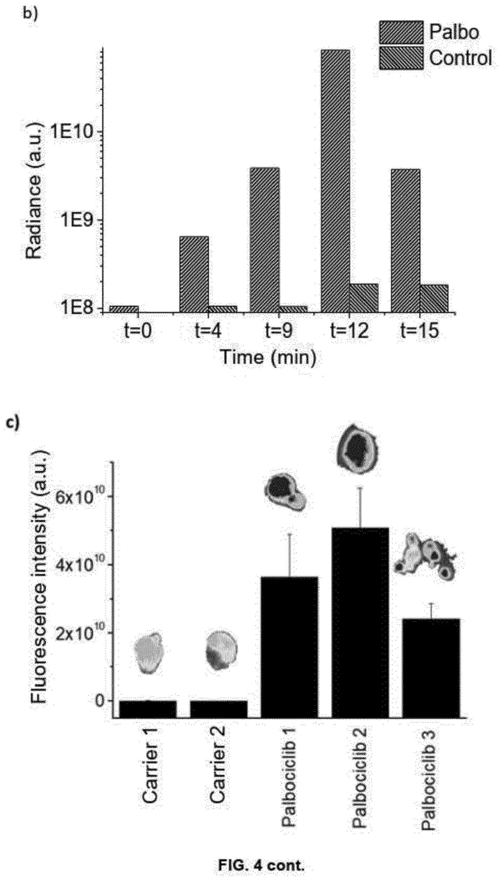

FIG. 4. a) *In vivo* images obtained by the *in vivo* imaging system (IVIS) technique, in which the mice were injected with 4T1 tumour cells (mouse mammary carcinoma). On the left, treatment with Palbociclib was carried out to obtain the transformation of the tumour cells into senescent cells and to stop tumour growth, while on the right (carrier), said treatment with Palbociclib was not performed, with the 4T1 cells remaining cancerous. b) Quantification of fluorophore Cy7 in the bladder over time. c) Quantification of fluorescence in urine with the IVIS technique after metabolisation of probe Cy7-Gal in the body. The female mice urinated after coming out from under anaesthesia due to the diuretic properties of probe Cy7-Gal.

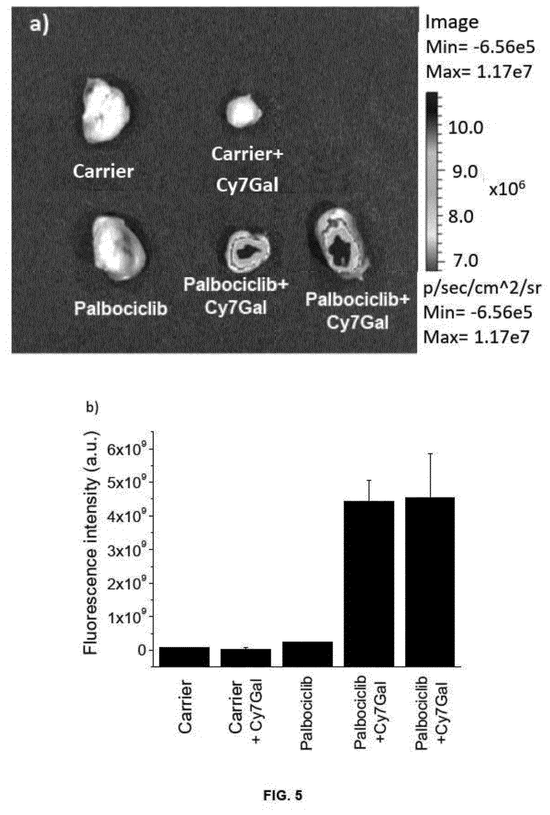

FIG. 5. a) *Ex vivo* images of the 4T1 cell tumours extracted after sacrificing the female mice in the different groups. b) Quantification of the fluorescence of Cy7 in the extracted tumours.

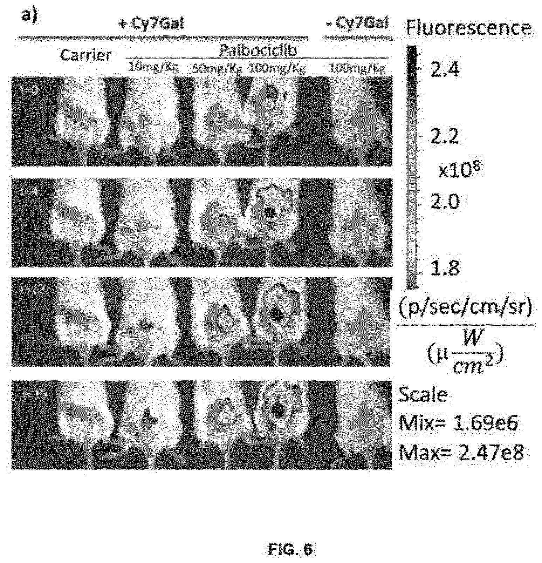

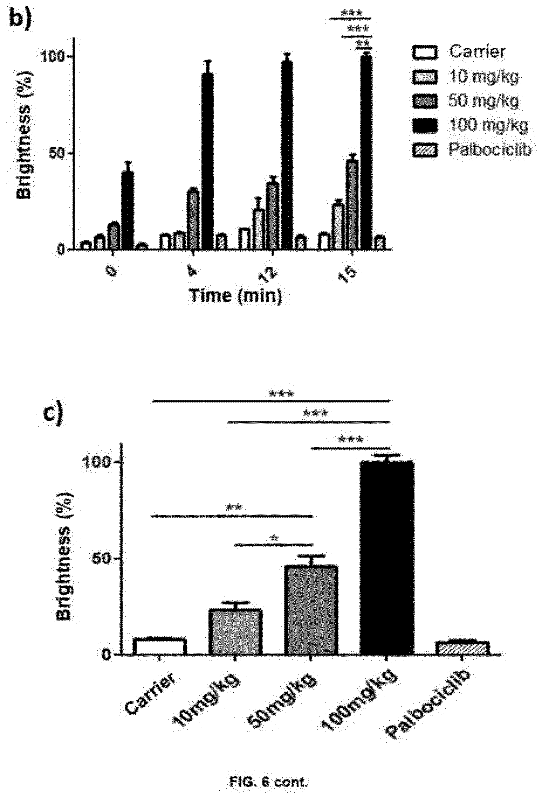

FIG. 6. a) Images obtained by the IVIS technique in which 4T1 cells (mouse mammary carcinoma) were injected. b) Quantification of the fluorescence signal in the bladder at different times and concentrations of Palbociclib. c) Extension of the fluorescence signal after 15 min for the different treatment groups.

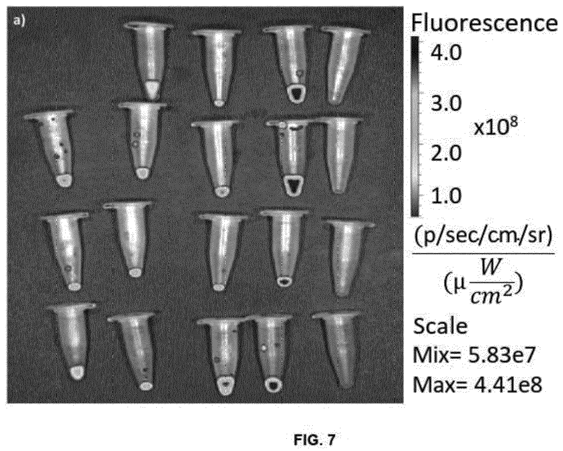

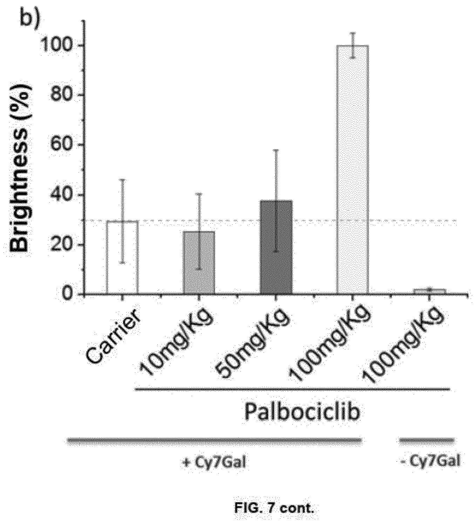

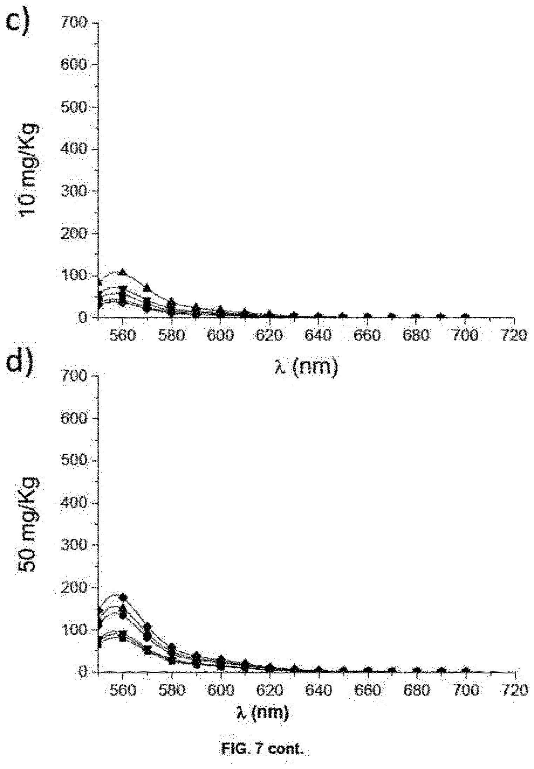

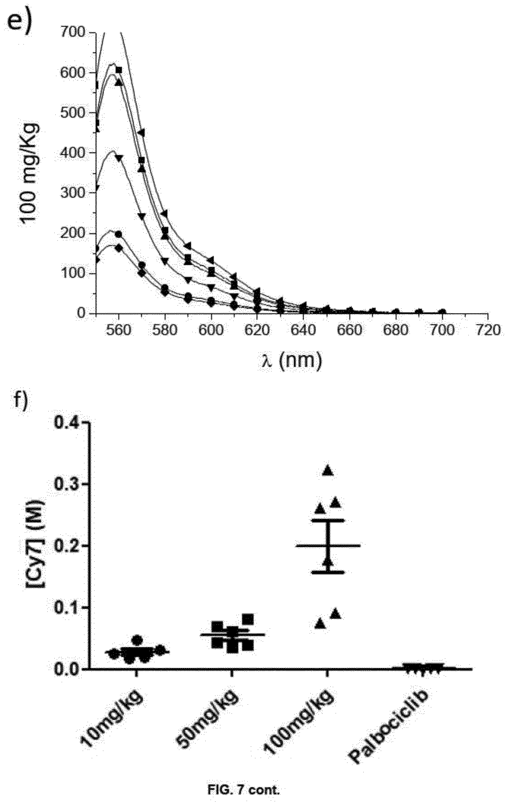

FIG. 7. a) IVIS images of the urine obtained for the different groups, from left to right the columns correspond with the following groups: not treated with Palbociclib, 10 mg/Kg of Palbociclib, 50 mg/Kg Palbociclib, 100 mg/Kg, all treated with the probe, and finally, 100 mg/kg Palbociclib not treated with probe Cy7-Gal. b) Quantification of fluorescence for the urine samples from the different groups with the IVIS technique. c) Fluorescence measurements obtained for the urine collected from the different groups. d) Fluorometric quantification of urine for the different groups.

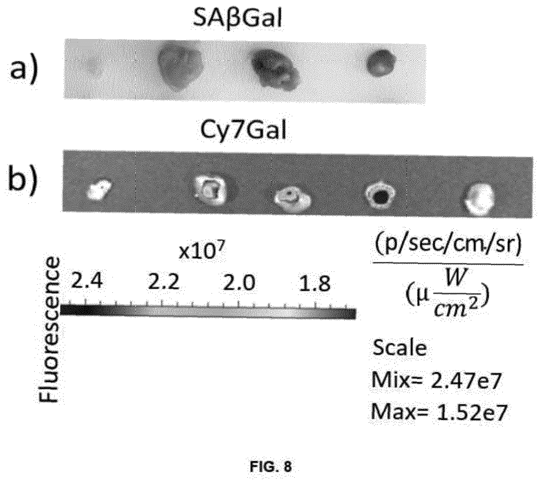

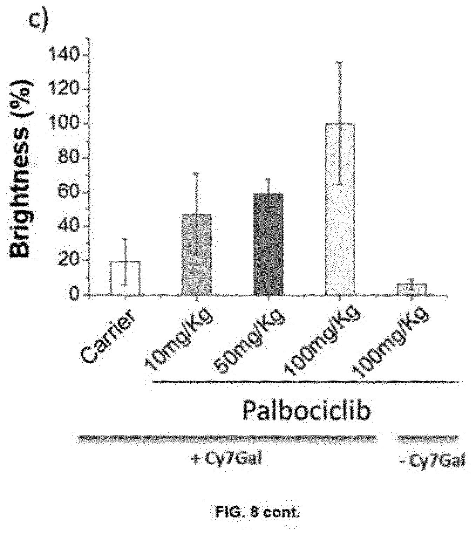

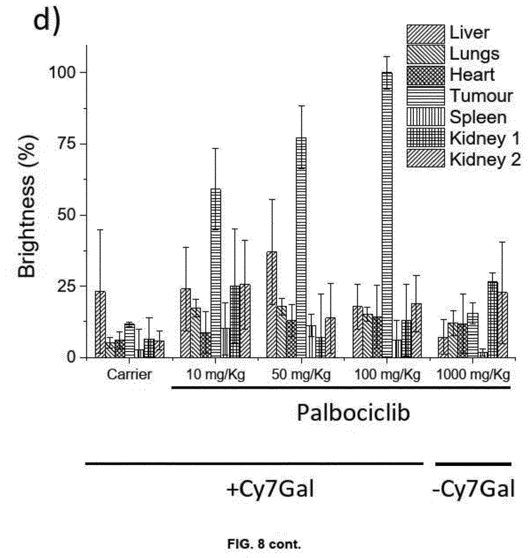

FIG. 8. a) *Ex vivo* images of the 4T1 cell tumours extracted after sacrificing the different groups, subsequently carrying out staining with X-gal, a commercial marker used to corroborate the existence of cellular senescence, where it is possible to carry out a semiquantitative determination based on the greenish-blue colouring that the tumour acquires. Observed from left to right are the tumours of the group not treated with Palbociclib and the tumours obtained from the groups treated with concentrations of 10 mg/Kg, 50 mg/kg and 100 mg/Kg of Palbociclib. b) Quantification by IVIS of the fluorescence signal for the different tumours shown above. c) Mean quantification of fluorescence corresponding to the hydrolysed probe of the tumours extracted in each group. d) Quantification by IVIS of the tumours and the organs that are most representative for the different groups, showing that a signal is only observed in senescent tumours, and no signal is observed in the different organs.

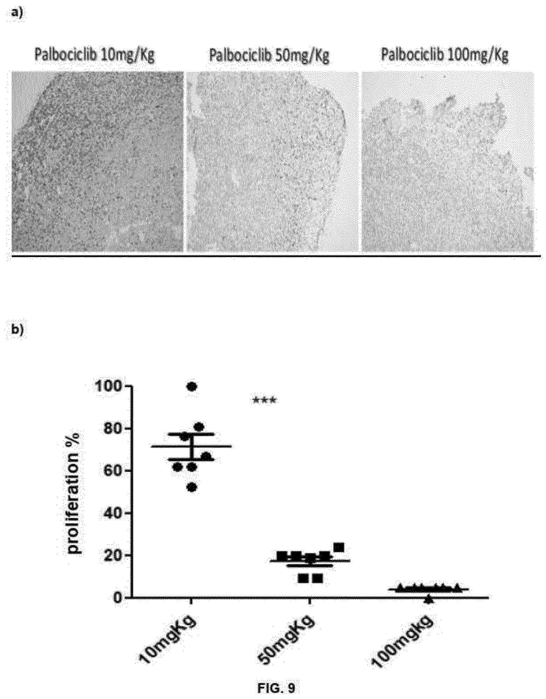

FIG. 9. a) Immunohistochemical quantification of the degree of senescence by means of staining proliferation biomarker Ki67. b) Quantification of biomarker Ki67, showing a drop in cell proliferation with the increase in the concentration of Palbociclib used.

### EXAMPLES

Next, the invention will be illustrated by means of assays carried out by the inventors that demonstrate the effectiveness of the product of the invention.

### Example 1: Synthesis of probe Cv7-Gal:

Probe Cy7-Gal was prepared according to following reaction scheme:

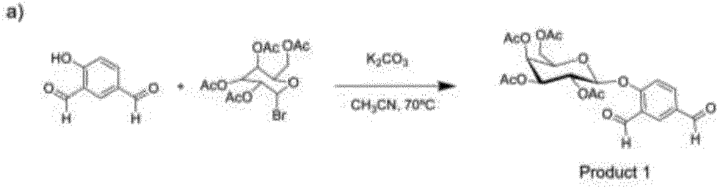

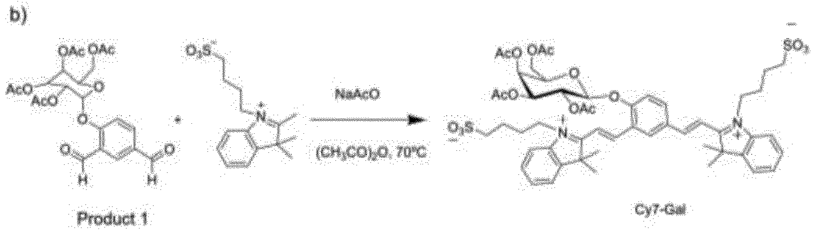

### Materials and methods:

Compound 5-Formylsalicylaldehyde was acquired from the company Tokyo Chemical Industry Co., Ltd. (TCI), while α-acetobromogalactose, potassium carbonate, the inert salt of 1-(4-sulfobutyl)-2,3,3-trimethylindolium), sodium acetate and acetic anhydride were acquired from Sigma-Aldrich. Anhydrous acetonitrile was purchased from the company Acros Organics B.V.B.A. Phosphate-buffered saline (PBS) was acquired from Sharlab S.L., The Dulbecco's Modified Eagle Medium (DMEM) and foetal bovine serum (FBS) were purchased from Gibco. Palbociclib was acquired from Selleckchem. Clear-bottom 96-well plates and cellTiter-Glo(R) Luminescent Cell for cellular viability assay were obtained from Promega. Senescence beta-Galactosidase KIT for X-gal staining was acquired from Werfrem. Anti-Ki67 antibody (ab833) was obtained from Abcam. Recombined human β-Galactosidase-1/GLB1 Protein (Human β-Gal) was obtained from R&D system a biotechne brand. 4T1 cells (mouse mammary carcinoma) were acquired from the American Type Culture Collection (ATCC). Female BALB/cByJ mice were acquired from Charles River laboratories, France.

A Tripletof T5600 mass spectrometer (ABSciex, USA) was used for the mass spectrometry. In the case of Nuclear Magnetic Resonance (NMR) spectroscopy for the characterisation of ¹H and ¹³C, Bruker FT-NMR Avance 400 (Ettlingen, Germany) spectrometre 300K was used, in which tetramethylsilane (TMS) was used as an internal standard. The fluorescence spectroscopy measurements were taken with a JASCO spectrofluorometer FP-8500. In the meantime, confocal fluorescence images of 4T1 cell cultures were acquired in a Leica TCS SP8 AOBS microscope and analysed using Image software. In turn, IVIS images were acquired from IVIS^{®} spectrum CT imaging equipment and analysed using the Caliper Life Sciences live image software both for the samples *in vivo* and in the case of organs and tumours *ex vivo.* The luminescence measurements for the viability assays were taken in a VICTOR Multilabel Plate Reader (Pelkin Elmer)

### Synthesis and characterisation:

The reagents 5-Formylsalicylaldehyde (75 mg, 0.5 mmol), α-acetobromogalactose (607 mg, 1.5 mmol) and potassium carbonate (400 mg, 4 mmol) were introduced in a roundbottom flask under argon atmosphere and dissolved in 30 ml of anhydrous acetonitrile. The reaction mixture was heated at 70 °C under stirring for 4 h, maintaining the inert atmosphere. After completing the reaction, the solvent was evaporated by means of reduced pressure. The reaction crude product was purified using a silica gel column and a hexane-ethyl acetate mixture (2:1 v/v) as an eluent. Product 1 was obtained as a clear greenish oil (230 mg, 0.46 mmol, 92.6 % yield).

In a second step, product **1** obtained in the previous step (90 mg, 0.17 mmol) was introduced together with 1-(4-sulfobutyl)-2,3,3-trimethylindolium salt (100 mg, 0.35 mmol) in a Schlenk tube under inert conditions. The solids were dissolved in 2 ml of acetic anhydride. The reaction is stirred at 70 °C for 4 h under an inert atmosphere. After that, the solvent is evaporated under reduced pressure. The reaction crude product is purified by means of reverse phase chromatography column using as an eluent a dichloromethane: methanol mixture (10:1 v/v) to obtain Cy7-Gal as a reddish brown solid (120 mg, 0.116 mmol, 68.1 % yield). ¹H NMR (400 MHz, CDCl₃) δ = 7.72 - 7.62 (m, 1H), 7.53 (dd, J = 7.5; 1.6 Hz, 1H), 7.49 - 7.43 (m, 2H), 7.42 - 7.32 (m, 2H), 7.00 (d, J = 7.3 Hz, 2H), 6.79 (t, J = 7.2 Hz, 1H), 6.69 (t, J = 7.3 Hz, 1H), 6.37 (d, J = 1.7 Hz, 1H), 5.69 (d, J = 8.3 Hz, 1H), 5.49 (d, J = 1.3 Hz, 1H), 5.42 (dd, J = 3.4; 0.9 Hz, 1H), 5.34 - 5.32 (m, 2H), 5.24 (s, 1H), 5.07 (dd, J = 10.4; 3.4 Hz, 1H), 4.17 - 4.04 (m, 4H), 3.48 (s, 4H), 2.91 (s, 4H), 2.03 (s, 12H), 1.93 (s, 4H), 1.82 (s, 4H), 1.58 (s, 12H).

¹³C- NMR (101 MHz, DMSO) δ =169.98 (1C), 169.88 (1C), 169.83 (1C), 169.55 (1C), 169.53 (1C), 169.51 (1C), 160.25 (1C), 160.01 (1C), 158.49 (1C), 142.26 (4C), 135.71 (1C), 131.03 (1C), 130.85 (1C), 129.31 (2C), 129.10 (1C), 127.74 (2C), 126.92 (2C), 122.89 (1C), 121.01 (2C), 115.67 (2C), 97.17 (1C), 73.73 (1C), 71.08 (1C), 68.35 (1C), 65.90 (1C), 63.53 (2C), 63.38 (2C), 61.51 (1C), 49.02 (2C), 36.20 (2C), 28.24 (2C), 24.39 (1C), 24.26 (1C), 24.09 (1C), 23.68 (1C), 20.53 (1C), 20.48 (1C), 20.34 (1C), 20.25 (1C). HRMS (high-resolution mass spectrometry): Calculated for C₅₂H₆₃N₂O₁₆S₂ (M+H⁺) 1035.3619 m/z; found 1035.3606 m/z (M+H⁺), 1051.3546 (M+H₂O-2H⁺).

### Confocal microscopy:

Mouse mammary carcinoma 4T1 cells were cultured in DMEM supplemented with 10 % FBS and incubated in 20 % O₂ and 5 % CO₂ at 37 °C. The cells were incubated with 5 µM of Palbociclib in DMEM for one week to induce the senescence process. Subsequently, the cells were seeded in a flat clear-bottom 96-well plate with a density of 6000 cells for senescent cells and 4000 in the control cells (without prior treatment with Palbociclib). After 24 hours, the cells were incubated with different concentrations of probe Cy7-Gal and its fluorophore (Cy7), with confocal microscopy images being acquired after 30 min of incubation. A more than 10-fold increase in fluorescence emission is observed between senescent cells treated with probe Cy7-Gal and untreated senescent cells (self-fluorescence signal). This increase in fluorescence emission is slightly higher between senescent cells and control cells when both have been incubated in the presence of probe Cy7-Gal, thereby providing high selectivity of the probe for detecting senescent cells.

### Fluorescence spectroscopy:

Urine fluorescence measurements were carried out by taking 5 µl of urine and diluting it in distilled water to 100 µl. The samples were obtained by exciting at 535 nm and collecting the absorption maximum at 566 nm. A correlation can be observed between the degree of senescence and the fluorescence intensity emitted by the urine corresponding to the presence of free fluorophore Cy7.

### Viability assays:

Mouse mammary carcinoma 4T1 cells were cultured in DMEM supplemented with 10 % FBS and incubated in 20 % O₂ and 5 % CO₂ at 37 °C. To obtain cellular senescence, the cells were incubated with 5 µM of Palbociclib in DMEM for one week. Subsequently, the cells were seeded in a flat clear-bottom 96-well plate with a density of 6000 cells for senescent cells and 4000 in the control cells. After 24 h, the cells were treated with different concentrations of probe Cy7-Gal and its fluorophore (Cy7). Viability measurements were obtained after 24 h of incubation using CellTiter-GLO Luminescent Cell Viability Assay as a developer. The viability assays showed that both probe Cy7-Gal and its fluorophore Cy7 exhibit minimum toxicity, even at much higher exposure times than those used in *in vivo* assays.

### In vivo assays:

The mice were kept in the Prince Phillip Research Centre (Centro de Investigación Príncipe Felipe (CIPF)) following the recommendations of the Federation of European Laboratory Animal Science Associations (FELASA). All the processes applied on the animals were approved by the Technical Research Committee for Animal Welfare (Comité Técnico de Investigación para el bienestar de los animals, CElyBA). 4T1 mammary tumours were generated using 4T1 cells: The cells were cultured in DMEM supplemented with 10 % FBS and penicillin-streptomycin. To generate the formation of mammary tumours, the cells were trypsinised and counted with a LUNA ^{™} automated cell counter and injected by subcutaneous route into the left mammary of 28- to 34-week-old female BALB/CBY mice at a concentration of 0.5·10⁻⁶ cells in a volume of 100 µl. The volume of the tumour was measured every two days with a gauge and calculated as V = (a x b2) / 2, wherein a is the longest and b is the shortest of two perpendicular diameters. The Palbociclib was administered through an oral probe daily for 7 days at different concentrations of 10 mg / kg, 50 mg / kg and 100 mg / kg dissolved in 50 mM sodium lactate at pH 5 in order to induce senescence. Subsequently, probe Cy7-Gal was administered at a concentration of 23.3 mg/ml in a volume of 100 µl. The mice were monitored for 15 minutes post-injection of the probe in the IVIS^{®} equipment following the accumulation of fluorophore Cy7 in the bladder after the hydrolysis of probe Cy7-Gal in the presence of senescent cells. The female mice urinated after coming out from under anaesthesia, at which time both probe Cy7-Gal and fluorophore Cy7 were expelled from the organism, favoured by the presence of the diuretic unit. The fluorescence of the urine samples for the different groups of mice was obtained directly using the IVIS technique and subsequently by means of fluorescence spectroscopy, corroborating the correspondence between the fluorescence emission of the urine samples and the degree of cellular senescence induced in different subjects, as well as the possible detection of the probe in the urinary tract allowing the *in vivo* measurement of cellular senescence. Subsequently, the mice were sacrificed and their organs rapidly extracted. To corroborate the presence of cellular senescence, tumours were stained with the commercial X-gal kit following the protocol described for such purpose; said staining allows semiquantification of senescence based on colouring, while carrying out in the same way the immunohistochemical staining of Ki67, a proliferation biomarker. The measurement of fluorescence in organs and tumours for the different groups of mice was performed with the IVIS technique.

## Claims

1. The compound, **characterised in that** it comprises:
- a signalling unit which consists of a fluorophore comprising at least one anionic group and
- a recognition unit which consists of a saccharide,
wherein the signalling unit is bound directly and covalently to the recognition unit.

2. The compound according to claim 1, wherein the saccharide comprises between 1 and 50 monosaccharides.

3. The compound according to claim 2, wherein the saccharide comprises between 1 and 20 monosaccharides.

4. The compound according to any of the preceding claims, wherein between 60 and 95% of the monosaccharide units are galactose, fucose and/or derivatives thereof.

5. The compound according to any of the preceding claims 1 to 3, the monosaccharides are galactose, fucose and/or derivatives thereof.

6. The compound according to any of claims 1 to 5, wherein the signalling unit comprises two anionic groups.

7. The compound according to any of claims 1 to 6, wherein the anionic groups are sulphonate, carboxylate, sulphate and/or halide groups.

8. The compound according to any of claims 1 to 7, wherein the fluorophore has a structure as defined below: 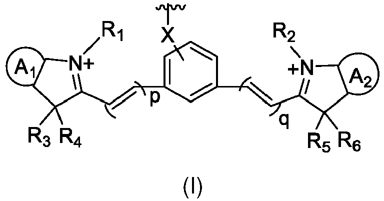 wherein A₁ and A₂ are independently aryl or heteroaryl groups,
*p* and *q* are independently an integer ranging between 1 and 4,
R₁ and R₂ are independently a -(CH₂)ₙ-Rₐ group, wherein:
Rₐ is an anion selected from the list consisting of: -SO₃⁻ -CO₂⁻, -SO₄⁻, halide,
n is an integer ranging between 0 and 8,
X is O, N, P or S, and
R₃, R₄, R₅, R₆ are selected independently from: C1-C8 alkyl, aryl and heteroaryl,
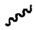 represents the point of the covalent bond with a carbon of a monosaccharide of the recognition unit.

9. The compound according to claim 8, wherein A1 and A2 are independently phenyl.

10. The compound according to claim 8 or 9, wherein p and q are independently 1.

11. The compound according to any of claims 8 to 10, wherein R1 and R2 are independently a -(CH2)n-SO3- or -(CH2)n-CO2- group, where n is an integer ranging between 0 and 8.

12. The compound according to any of claims 8 to 11, wherein X is O.

13. The compound according to any of claims 8 to 12, wherein R3, R4, R5, R6 are independently methyl.

14. The compound according to claim 8, wherein the fluorophore has the following formula:

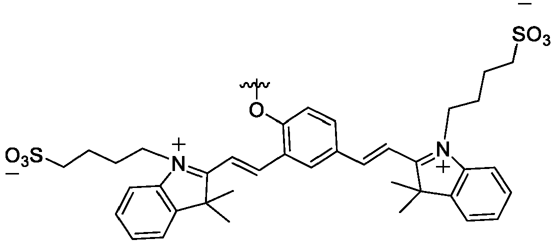

15. The compound according to claim 8, exhibiting the following formula: 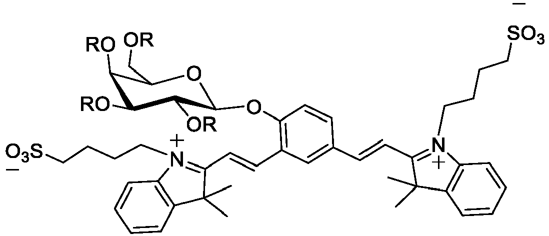 wherein R is H or acetyl.

16. A composition comprising the compound described in any of the preceding claims 1 to 15 and a pharmaceutically acceptable carrier

17. Use of the compound described in any of claims 1 to 15, or of the composition described in claim 16, as a probe for detecting senescent cells in a urine sample from a subject in whom said compound has been administered.
